# EUROPEAN PATENT APPLICATION

(11) **EP 3 015 055 A1**
(43) Date of publication of application: **04.05.2016**
(21) Application number: 14191154.5
(22) Date of filing: 30.10.2014
(51) Int. Cl.: A61B 5/00

(54) **System for monitoring a driver**

(71) Applicant: Comftech S.r.L., 20900 Monza (MB) (IT)
(72) Inventor: Andreoni, Giuseppe, 20835 Muggiò (MB) (IT); Moltani, Lara Alessia Laura, 20900 Monza (MB) (IT)
(74) Representative: Ciceri, Fabio

(57) **Abstract**

The present disclosure relates to a system (1) for monitoring physiological parameters of a vehicle driver/rider. The system (1) comprises a steering member (2) of the vehicle, having a first processing unit (21) configured to provide a plurality of data values (D) of the physiological parameters of the driver/rider, a sensorized glove (3) having such a shape as to cover a hand of the driver/rider, as he/she drives/rides the vehicle, the sensorized glove (2) comprises at least one first textile sensor (31) which is configured to sense a first physical quantity of the physiological parameters of the driver/rider, to thereby generate a first signal (S1), the sensorized glove (3) is equipped with a second processing unit (33), the second processing unit (33) is in signal communication with the first textile sensor (31) to receive and process the first signal (S1) to thereby generate a third signal (S3), the steering member (2) comprises at least one conductive element (22), the first processing unit (21) and the second processing unit (23) are in signal communication with each other via the conductive element (22), the first processing unit (21) is configured to receive and process the third signal (S3), to thereby generate the plurality of data values (D) of the physiological parameters of the driver/rider.

## Description

### FIELD OF THE INVENTION

The present disclosure relates to a system for monitoring the physiological parameters of a vehicle driver/rider.

### Discussion of the related art

It is known in the art to use a system for measuring physiological parameters, such as biomedical and/or biometric signals. These signals comprise electrophysiological signals, such as electrocardiogram (ECG), electroencephalogram (EEG), electromyography (EMG), electrodermal activity (EDA), galvanic skin response (GSR), movement of the body or of some of its anatomical regions and respiratory activity. Biomedical sensors have been recently developed (e.g. for measuring electrophysiological signals), which are adapted to be worn for monitoring patients at home, hospital and in daily life, or for monitoring athletes during physical exercise and sports. Sensors with one or more electrodes having conductive textile fibers provide a number of advantages, including flexibility, comfort and easy incorporation into a garment.

Interlaced or tessellated conductive fibers in textile structures form textile electrodes (or sensors) which allow measurement and/or monitoring of electric potentials, when they are connected to a measurement system.

There are also known sensors (e.g. metal plates), which are attached to handlebars or handles of exercise equipment, such as exercise bikes or treadmills, for sensing heartbeat during physical exercise and sports training. These sensors can measure the heartbeat rate when they contact the wrist of an individual.

### Prior art problem

Prior art systems for measuring physiological parameters are not suitable for applications in vehicles and motorized means of transport (e.g. cars, motorcycles, helicopters) and non-motorized ones (e.g. bicycles). Prior art sensors attached to the handlebar of a motorcycle or the steering wheel of a car do not afford continuous sensing of the physiological parameters of the driver. This is because the driver frequently moves his/her hands on the wheel as he/she drives and his/her wrists do not continuously and stably contact the sensorized portion of the wheel. Furthermore, especially in the case of bicycle and motorcycle handlebars, riders often use gloves to protect themselves from the cold in Winter and from rubbing during sports activities or driving. Therefore, the wrist of the rider does not continuously contact the sensor, and physiological parameter sensing is thus prevented.

### SUMMARY OF INVENTION

The object of the subject invention is to provide a system for continuous sensing of the physiological parameters of a vehicle driver/rider.

Therefore, a further object of the invention is to provide a system for sensing the physiological parameters of a driver and the contact pressure when the driver holds a steering member of the vehicle.

### Advantages of the invention

In one embodiment a system may be provided for sensing the physiological parameters of a driver/rider without affecting, disturbing or altering the driving/riding experience.

In a further embodiment, a system may be provided for sensing the physiological parameters of the driver/rider and the contact pressure when the driver/rider holds or handles a steering member. This allows detection of which steering member is used by the driver/rider in real time.

In another embodiment, a system may be provided for sensing the parameters of the driver/rider and vehicle attitude parameters.

### BRIEF DESCRIPTION Of THE DRAWINGS

The characteristics and advantages of the present disclosure will appear from the following detailed description of a possible practical embodiment, illustrated as a non-limiting example in the set of drawings, in which:

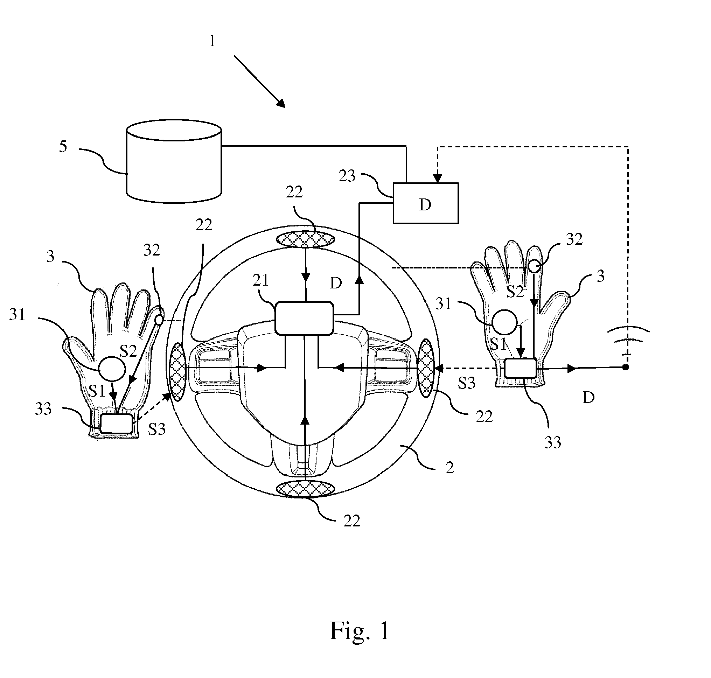

- Figure 1 shows a schematic view of a system for monitoring the physiological parameters of the present invention,

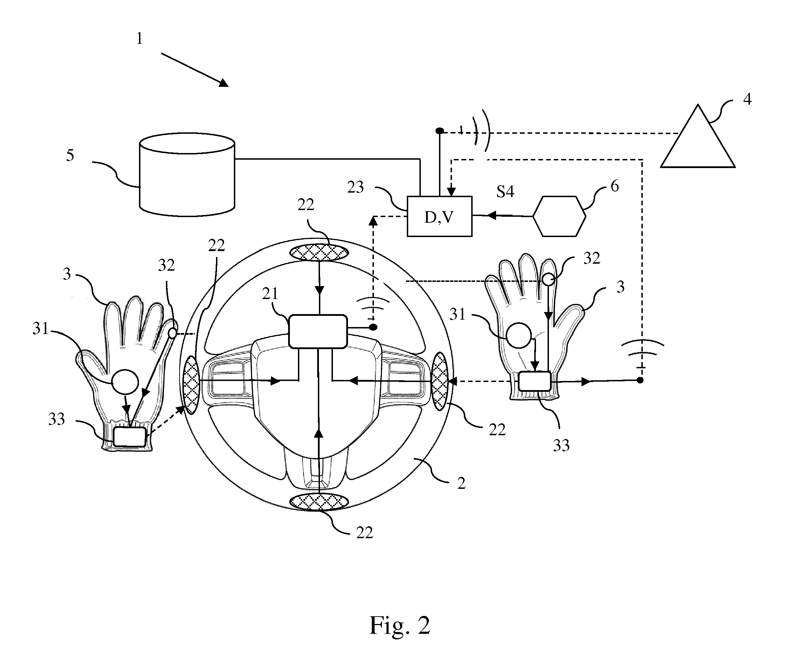

- Figure 2 shows a schematic view of a different embodiment of the system for monitoring the physiological parameters of Figure 1,

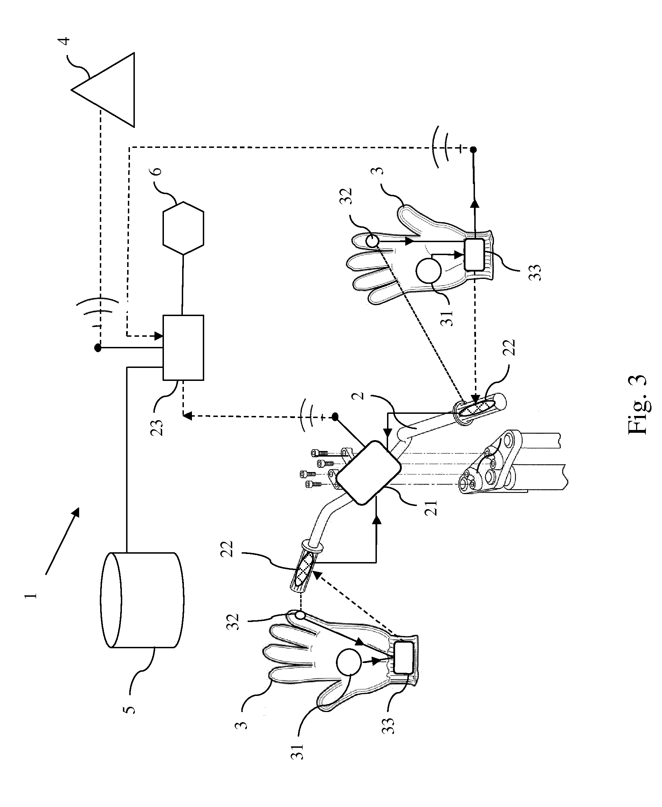

- Figure 3 shows a schematic view of a further embodiment of the system for monitoring physiological parameters.

### DETAILED DESCRIPTION

Even when this is not expressly stated, the individual features as described with reference to the particular embodiments shall be intended as auxiliary to and/or interchangeable with other features described with reference to other exemplary embodiments.

The present invention relates to a system 1 for monitoring the physiological parameters of a vehicle driver/rider.

For brevity, reference will be made hereinafter without limitation to a vehicle to intend any means of transport by land or air, either motorized (e.g. cars, scooters, motorcycles, boats, helicopters, airplanes) or non-motorized (bicycles, push scooters) and driven by a driver/rider.

The system 1 comprises a steering member 2 of the vehicle.

For brevity, reference will be made hereinafter without limitation to a steering member 2 to intend any means for controlling or steering a motorized or non-motorized aerial or terrestrial vehicle.

Possible examples of a steering member 2 are the handlebar of a bicycle or a scooter, the steering wheel or the gear lever of a car, the brake or clutch wheel lever, the yoke (control stick) of an airplane.

This steering member 2 has a first processing unit 21 incorporated therein. The first processing unit 21 is configured to provide a plurality of data values D of the physiological parameters of the driver/rider.

For brevity, reference will be made hereinafter without limitation to the plurality of data values D of the physiological parameters of the driver/rider to intend a plurality of data values reflecting, for instance, the heartbeat of the driver/rider.

The plurality of data values D is configured to be accessed and displayed by a user using any computer, PC, tablet PC and smartphone having a graphic interface.

The system 1 further comprises a sensorized glove 3 which is shaped to cover a hand of the driver/rider when the driver/rider drives/rides the vehicle. The sensorized glove 2 comprises at least one first textile sensor 31. Each first textile sensor 31 is configured to sense a first physical quantity of the physiological parameters of the driver/rider, to generate a first signal S1. Preferably, the first textile sensor 31 is a sensor for measuring electrical physiological signals (e.g. electrical heartbeat signals).

The sensorized glove 3 comprises a second processing unit 33 incorporated therein. The second processing unit 33 is in signal communication with the first textile sensor 31 to receive and process the first signal S 1, to thereby generate a third signal S3.

The steering member 2 comprises at least one conductive element 22.

The first processing unit 21 and the second processing unit 23 are in signal communication with each other via the conductive element 22. The first processing unit 21 is configured to receive and process the third signal S3, to thereby generate the plurality of data values D of the physiological parameters of the driver/rider. Preferably, the conductive element 22 is a textile conductive element or a metal (e.g. steel) plate incorporated in the steering member 2.

Preferably, the conductive element/s 22 are placed at the gripper portions of the steering member 2.

According to a preferred embodiment, the sensorized glove 3 comprises at least one second textile sensor 32.

Each second textile sensor 32 is configured to sense a second physical quantity reflecting the intensity of pressure contact between the sensorized glove 3 and the steering member 2, to generate a second signal S2.

The second processing unit 33 is in signal communication with the first textile sensor 31 and the second textile sensor 32 to receive and process the first signal S1 and the second signal S2, to thereby generate the third signal S3.

The plurality of data values D reflect the physiological parameters of the driver/rider and the intensity of contact pressure between the sensorized glove 3 and the steering member 2.

In other words, with the first textile sensor 31 the physiological parameters (e.g. the heartbeat) of the driver/user may be monitored.

Advantageously, with the second textile sensor 32 information may be obtained about the position of the hands of the driver/rider on the steering member 2, and also about the intensity with which the driver/rider holds the steering member 2, and the times at which this occurs.

Advantageously, the sensorized glove 3 allows heartbeat measurement and assessment of contact pressure by the hand and the individual fingers on the steering wheel/handlebar and/or other controls (e.g. brake/clutch/gear lever). Thus, the system 1 can also check whether these steering members 2 are used and actuated.

According to a preferred arrangement, the conductive element 22 is configured to receive and send the third signal S3 to the first processing unit 21 when the conductive element 22 directly contacts at least one conductive portion of the sensorized glove 3. The conductive portion of the sensorized glove 3 is in signal communication with the second processing unit 33.

In other words, the conductive portion of the sensorized glove 3 transmits the third signal S3 to the conductive element 22 when they come to direct (and electric) contact, i.e. when the hand of the driver/rider (covered by the sensorized glove 3) holds the steering member 2.

According to a preferred embodiment, the system 1 comprises a third processing unit 23 associated with a database 5. The third processing unit 23 is in signal communication with the first processing unit 21 to receive the plurality of data values

Preferably, the third processing unit is incorporated in the steering member 2. The third processing unit 23 is in signal communication with the first processing unit 21 via wires or a radio-frequency protocol (e.g. Bluetooth, Wi-Fi).

According to a preferred arrangement, the third processing unit 23 is configured to send the plurality of data values D to a fourth processing unit 4 outside the vehicle, via a radio-frequency protocol (e.g. Bluetooth).

Preferably, the first processing unit 21 and/or the second processing unit 33 and/or the third processing unit 23 and/or the fourth processing unit 4 are central processing units (i.e. CPUs), such as microprocessors.

More preferably, the second processing unit 33 can perform bandpass filtering on the the first signal S1 and/or the second signal S2, to thereby generate the third signal S3.

According to a preferred arrangement, the system comprises at least one third sensor 6 which is configured to sense a third physical quantity reflecting vehicle attitude, to generate a fourth signal S4.

The third processing unit 23 is in signal communication with the third sensor 6. The third processing unit 23 is configured to receive and process the fourth signal S4 to generate a plurality of vehicle data values V and store them into the database 5. The plurality of vehicle data values V reflects the attitude of the vehicle.

For brevity, reference will be made hereinafter without limitation to the attitude of the vehicle to intend parameters of speed, acceleration, inclination in the three spatial directions and possibly abrupt changes to these parameters (e.g. in case of accident).

According to a preferred embodiment, the third processing unit 23 is configured to send the plurality of vehicle data values V to the fourth processing unit 4, via a radio-frequency protocol (e.g. Bluetooth).

According to a preferred arrangement, the second processing unit 33 is configured to receive and process the first signal S1 and the second signal S2 to generate the plurality of data values D. The second processing unit 33 is configured to send the plurality of data values D to the third processing unit 23, via a radio-frequency protocol (e.g. Bluetooth, Wi-Fi).

Those skilled in the art will obviously appreciate that a number of changes and variants as described above may be made to fulfill particular requirements, without departure from the scope of the invention, as defined in the following claims.

## Claims

1. A system (1) for monitoring physiological parameters of a vehicle driver/rider, which comprises a steering member (2) of said vehicle, having a first processing unit (21) configured to provide a first plurality of data values (D) of said physiological parameters of said driver/rider and at least one conductive element (22), **characterized in that** it comprises:
- a sensorized glove (3) having such a shape as to cover a hand of said driver/rider, said sensorized glove (2) comprising:
- at least one first textile sensor (31) which is configured to sense a first physical quantity of said physiological parameters of the driver/rider, to generate a first signal (S1),
- a second processing unit (33) in signal communication with said first textile sensor (31) to receive and process said first signal (S1), to thereby generate a third signal (S3),
- said first processing unit (21) and said second processing unit (23) being in signal communication with each other via said conductive element (22),
- said first processing unit (21) being configured to receive and process said third signal (S3), to thereby generate said plurality of data values (D) of said physiological parameters of said driver/rider.

2. A system (1) as claimed in claim 1, wherein:
- said sensorized glove (3) comprises at least one second textile sensor (32) which is configured to sense a second physical quantity reflecting the intensity of pressure contact between said sensorized glove (3) and said steering member (2), to thereby generate a second signal (S2),
- said second processing unit (33) is in signal communication with said first textile sensor (31) and said second textile sensor (32) to receive and process said first signal (S1) and said second signal (S2), to thereby generate said third signal (S3),
- said plurality of data values (D) reflect said physiological parameters of said driver/rider and the intensity of contact pressure between said sensorized glove (3) and said steering member (2).

3. A system as claimed in claim 1 or 2, wherein:
- said conductive element (22) is configured to receive and send said third signal (S3) to said first processing unit (21) when said conductive element (22) directly contacts at least one conductive portion of said sensorized glove (3), said conductive portion of said sensorized glove (3) is in signal communication with said second processing unit (33).

4. A system (1) as claimed in any of the preceding claims, comprising:
- a third processing unit (23) associated with a database (5), said third processing unit (23) is in signal communication with said first processing unit (21) to receive and store said plurality of data values (D) into said database (5).

5. A system (1) as claimed in claim 4, wherein
- said third processing unit (23) is configured to send said plurality of data values (D) to a fourth processing unit (4) outside said vehicle, via a radio-frequency protocol.

6. A system (1) as claimed in claim 4 or 5, comprising
- at least one third sensor (6) which is configured to sense a third physical quantity reflecting the attitude of said vehicle, to thereby generate a fourth signal (S4),
- said third processing unit (23) is in signal communication with said third sensor (6) and is configured to receive and process said fourth signal (S4) to generate and store a second plurality of vehicle data values (V) into said database (5), said plurality of vehicle data values (V) reflecting the attitude of said vehicle.

7. A system (1) as claimed in claim 6, wherein
- said third processing unit (23) is configured to send said plurality of vehicle data values (V) to said fourth processing unit (4), via a radio-frequency protocol.

8. A system (1) as claimed in any of claims 4 to 7, wherein
- said second processing unit (33) is configured to receive and process said first signal (S1) and said second signal (S2) to generate said plurality of data values (D),
- said second processing unit (33) is configured to send said plurality of data values (D) to said third processing unit (23), via a radio-frequency protocol.

9. A system (1) as claimed in any of the preceding claims, wherein said conductive element (22) is a textile conductive element or a metal plate incorporated in said steering member (2).
